# EUROPEAN PATENT APPLICATION

(11) **EP 2 653 535 A1**
(43) Date of publication of application: **23.10.2013**
(21) Application number: 11849759.3
(22) Date of filing: 07.11.2011
(51) Int. Cl.: C12N 5/074, C12N 5/10, C12N 5/071, C01D 15/10, C01D 15/08, C01D 15/06, C01D 15/04, C01D 15/00

(54) **METHOD FOR PREPARING INDUCED PLURIPOTENT STEM CELLS AND CULTURE MEDIUM USED FOR PREPARING INDUCED PLURIPOTENT STEM CELLS**

(30) Priority: 16.12.2010 CN 201010594842
(71) Applicant: Shanghai Institute Materia Medica, Chinese Academy Of Sciences, Pudong, Shanghai 201203 (CN)
(72) Inventor: XIE, Xin, Pudong Shanghai 201203 (CN); WANG, Quan, Pudong Shanghai 201203 (CN)
(74) Representative: Grünecker, Kinkeldey, Stockmair & Schwanhäusser
(86) International application number: PCT/CN2011/001875
(87) International publication number: WO 2012/079278

(57) **Abstract**

A method for preparing induced pluripotent stem (iPS) cells, which comprises steps as follows: step 1, introducing one or more stem cell pluripotency factors into somatic cells; step 2, culturing the somatic cells, into which the stem cell pluripotency factor has been introduced in the Step 1, by using medium supplemented with lithium salt; and step 3, identifying and characterizing the induced pluripotent stem cells. Furthermore, there provided a medium for preparing induced pluripotent stem cells, which comprising lithium salt. The medium supplemented with lithium salt is used for efficiently inducing pluripotent stem cells. Lithium salt is able to increase the production efficiency of mouse iPS cells by 5-60 times. The present method for inducing iPS cells is in favor of improving the safety of iPS technique and application of iPS cells in regenerative medicine.

<110>  SHANGHAI INSTITUTE OF MATERIA MEDICA, CHINESE ACADEMY
       OF SCIENCES

<120>  METHOD FOR PREPARING INDUCED PLURIPOTENT STEM CELLS
       AND MEDIUM FOR PREPARING INDUCED PLURIPOTENT STEM CELLS

<130>  EP88302FZSEpau

<140>  EP11849759.3
<141>  2011-11-07

<150>  PCT/CN2011/001875
<151>  2011-11-07

<150>  2010-10594842.6
<151>  2010-12-16

<160>  16

<170>  PatentIn version 3.3

<210>  1
<211>  20
<212>  DNA
<213>  Artificial

<220>
<223>  endogenous-Oct4 F

<400>  1
taggtgagcc gtctttccac                                                    20


<210>  2
<211>  20
<212>  DNA
<213>  Artificial

<220>
<223>  endogenous-Oct4 R

<400>  2
gcttagccag gttcgaggat                                                    20


<210>  3
<211>  20
<212>  DNA
<213>  Artificial

<220>
<223>  Nanog F

<400>  3
ctcaagtcct gaggctgaca                                                    20


<210>  4
<211>  20
<212>  DNA
<213>  Artificial

<220>
<223>  Nanog R

<400>  4
tgaaacctgt ccttgagtgc                                                    20


<210>  5
<211>  20
<212>  DNA
<213>  Artificial

<220>
<223>  endogenous-Sox2 F

<400>  5
agggctggga gaaagaagag                                                    20


<210>  6
<211>  20
<212>  DNA
<213>  Artificial

<220>
<223>  endogenous-Sox2 R

<400>  6
ccgcgattgt tgtgattagt                                                    20


<210>  7
<211>  18
<212>  DNA
<213>  Artificial

<220>
<223>  Rex1 F

<400>  7
gacgaagcaa gagaagag                                                      18


<210>  8
<211>  19
<212>  DNA
<213>  Artificial

<220>
<223>  Rex1 R

<400>  8
cgataagaca ccacagtac                                                     19

<210>  9
<211>  20
<212>  DNA
<213>  Artificial

<220>
<223>  exogenous-Sox2 F

<400>  9
gggtggacca tcctctagac                                                   20


<210>  10
<211>  18
<212>  DNA
<213>  Artificial

<220>
<223>  exogenous-Sox2 R

<400>  10
gggctgttct tctggttg                                                     18


<210>  11
<211>  20
<212>  DNA
<213>  Artificial

<220>
<223>  exogenous-Klf4 F

<400>  11
gggtggacca tcctctagac                                                   20


<210>  12
<211>  20
<212>  DNA
<213>  Artificial

<220>
<223>  exogenous-Klf4 R

<400>  12
gctggacgca gtgtcttctc                                                   20


<210>  13
<211>  20
<212>  DNA
<213>  Artificial

<220>
<223>  exogenous-cMyc F

<400>  13
gggtggacca tcctctagac                                                   20


<210>  14
<211>  20
<212>  DNA
<213>  Artificial

<220>
<223>  exogenous-cMyc R

<400>  14
cctcgtcgca gatgaaatag                                                   20

<210>  15
<211>  17
<212>  DNA
<213>  Artificial

<220>
<223>  exogenous-Oct4 F

<400>  15
gcttggatac acgccgc                                                      17


<210>  16
<211>  20
<212>  DNA
<213>  Artificial

<220>
<223>  exogenous-Oct4 R

<400>  16
ttcatgtcct gggactcctc                                                   20

## Description

### TECHNICAL FIELD

The present invention relates to the field of pharmacy and biotechnology, and more specifically to a method for preparing induced pluripotent stem cells and a medium for preparing induced pluripotent stem cells.

### BACKGROUND ART

Embryonic stem cells originate from the inner cell masses of early embryos, and are capable of self-renewal, maintaining pluripotency, and differentiating into cells of three germ layers while cultured *in vitro.* With the successful establishment of mouse embryonic stem cells in 1981 and human embryonic stem cells in 1998 [1, 2], research in regenerative medicine really started. Embryonic stem cells have been proposed to be mainly applied to transplantation treatment, serving as the starting cells in the *in vitro* culture and can be directionally differentiated into cells, tissues or organs used for clinical transplantation. For many specific cell types (for example, nerve cells, myocardial cells, etc.), various differentiation methods have already been established, based on the studies on the mechanisms of self-renewal and directional differentiation. These studies brought hopes for the treatment of Parkinson's disease, senile dementia, myocardial damage, diabetes, cirrhosis and other diseases. However, many technical problems and ethical issues have to be solved before embryonic stem cells can be transferred from in vitro studies towards clinical applications, for example, how to obtain immune rejection-free embryonic stem cells; or how to get patient-derived embryonic stem cells, and so on.

Yamanaka et al. first reported in 2006 that mouse fibroblasts could be de-differentiated into cells, which were named induced pluripotent stem (iPS) cells, with characteristics similar to embryonic stem cells by overexpressing four transcription factors (Oct4, Sox2, Klf4 and c-Myc) [3]. In this *Cell* article, researchers initially selected 24 genes associated with signal regulation of embryonic stem cells as candidate genes and transduced the mouse fibroblasts with retroviruses carrying these genes, and the pluripotency of iPS cells was characterized by cell morphology, expression of embryonic stem cell-specific genes, teratoma formation and so on. Eventually the 24 candidate genes were reduced to four transcription factors of Oct4, Sox2, Klf4 and c-Myc. Although the researchers did not get iPS cell-contributed chimera mice in this article, the approach in which stem cells were directly obtained from differentiated cells had brought conceptual innovation for the field of embryonic stem cells research and even the field of life sciences research. The emergence of the iPS technology also stirred great interest in the biomedical applications of iPS cells, because we can obtain patient-specific iPS cells by using the patient's somatic cells as sources, and these iPS cells can differentiate into functional cells, tissues or organs which can be used for disease treatment. Immuno-compatibility issues and ethical concerns can be avoided by means of such applications.

In 2007, three different laboratories published articles and reported that germline-competent mouse iPS cells were obtained [4-6]. After this, several laboratories reported that human iPS cells were obtained [7, 8]. In 2009, researchers obtained full-iPS mice with reproduction capacity by tetraploid complementation assay for the first time, demonstrating the totipotency of iPS cells [9]. IPS, as a technology of generating pluripotent stem cells, has enormous application value in the treatment of clinical disease. Unlike traditional methods for obtaining stem cells such as nuclear transplantation, cell fusion, etc., iPS cells can be obtained without being dependent on human oocytes or human embryonic stem cells, and immunological rejection can be avoided by reprogramming somatic cells to iPS cells so that autologous transplantation has become more feasible. On the other hand, iPS cells are also very important in the mechanism studies of self-renewal and signal regulation of stem cells as well as many diseases.

However, there are still two major obstacles for the clinical application of iPS cells, i.e., the safety concern and low induction efficiency. As for biosafety, the exogenous genes overexpressed in the initial experimental system included two oncogenes (c-Myc and Klf-4), and the exogenous genes were introduced by means of retroviral infection, thus multiple copies of viral genes could be inserted into the genome, which might lead to gene mutations and carcinogenesis. Therefore, researchers were committed to optimize the iPS technology, for example, by reducing the number of transcription factors [10, 11], or by using the gene transfection method without viral integration into genome [12], as well as by directly adding membrane-permeable transcription factor proteins to induce reprogramming [13], to improve the safety of its application,. However, these methods led to even lower efficiency in the induction of iPS cells. At present, the efficiency of iPS cells generation is generally less than 1%, which composes a major obstacle to the development and application of iPS cells. Therefore, researchers are looking for the culture conditions, methods or small molecule compounds to improve the induction efficiency of iPS cells and to push forward the basic research and clinical application of iPS cells.

### DISCLOSURE OF THE INVENTION

Therefore, the present inventors had made a variety of extensive and intensive studies in view of the problems existing in the prior art, and completed the invention.

In order to improve the generation efficiency of the iPS cells, the invention provides a method for preparing the induced pluripotent stem cells, which comprises steps as follows:
step 1: introducing one or more stem cell pluripotency factors into somatic cells;
step 2: culturing the somatic cells, into which the stem cell pluripotency factors have been introduced in the step 1, by using medium supplemented with lithium salt; and
step 3: identifying and characterizing the induced pluripotent stem cell clones.

Preferably, the method further comprises a step of introducing a reporter gene into the somatic cells, so as to indicate the generation of the induced pluripotent stem cells and production efficiency thereof via the reporter gene. More preferably the reporter gene is Oct4-GFP or Nanog-GFP, and most preferably is Oct4-GFP.

In the step 1, it is preferable that the cDNA of the stem cell pluripotency factor(s) is/are introduced into mouse embryonic fibroblasts by means of viral infection.

In the step 1, preferably, the stem cell pluripotency factor(s) may be one or more selected from the group consisting of Oct4, Sox2, Sox1, Klf4, Klf2, K1f5, Nanog, c-Myc, L-Myc, N-Myc, Lin28 and Esrrb. More preferably, the stem cell pluripotency factor(s) is/are one or more selected from the group consisting of Oct4, Sox2, Klf4 and c-Myc. Most preferably, the stem cell pluripotency factor(s) is/are one or more selected from the group consisting of Oct4, Sox2 and Klf4.

Preferably, in the step 2, the lithium salt(s) is/are selected from the group consisting of lithium chloride, lithium carbonate, lithium acetate, lithium bromide, lithium aspartate, lithium γ-linolenatₑ, lithium heparin, lithium sulfate, lithium nitrate, and other chemicals containing lithium ion.

More preferably, in the step 2, the lithium salt is lithium chloride.

Preferably, the concentration of the lithium salt(s) is from 0.1 mM to 40 mM. More preferably, the concentration of the lithium salt(s) is from 0.5 mM to 20 mM. Further more preferably, the concentration of the lithium salt(s) is from 1 mM to 10 mM. Even further more preferably, the concentration of the lithium salt(s) is from 5 mM to 10 mM. Most preferably, the concentration of the lithium salt(s) is 10 mM.

The medium used in the step 2 may be DMEM (Dulbcco's Modified Eagle's Medium) supplemented with 15% fetal bovine serum, 1000U/mL leukaemia inhibitory factor (LIF), L-glutamine, non-essential amino acid(s), penicillin/streptomycin and β-mercaptoethanol (mES medium), and Knockout DMEM supplemented with 15% serum replacement, 1000U/mL leukaemia inhibitory factor (LIF), L-glutamine, non-essential amino acid, penicillin/streptomycin and β-mercaptoethanol (KSR medium).

Preferably, the step 2 specifically comprises procedures as follows: mouse embryonic fibroblasts, into which four factors of Oct4, Sox2, Klf4 and c-Myc or three factors of Oct4, Sox2 and Klf4 have been introduced in the step 1, are trypsinized, seeded onto feeder cells and cultured by using the mES medium on the 2^{nd} day of viral infection, on the 3^{th} day the medium is replaced with the mES medium supplemented with lithium salt, on the 6^{th} day the medium is replaced with the KSR medium supplemented with lithium salt; and on the 8^{th} day the medium is replaced with the KSR medium, and
colonies with excellent stem cell-like morphology or Oct4-GFP⁺ colonies are selected and used for passage.

The term "colonies with excellent stem cell-like morphology" refer to the colonies wherein the cells exhibit similar morphology compared to that of mouse embryonic stem cells. The term "Oct4-GFP⁺ colonies" refers to the transgenic Oct4-GFP reporter gene positive colonies. Oct4 is the embryonic stem cell-specific gene and the expression of Oct4 indicates that the mouse embryonic fibroblasts have been reprogrammed to pluripotent stem cells in a relatively accurate way.

In the step 3, the methods for characterization of the induced pluripotent stem cell colonies include AP staining, detection of endogenous Oct4 expression, detection of pluripotency marker expression with quantitative PCR, detection of the silencing of the exogenous viral genes, teratoma formation, chimera formation, and detection of germline transmission.

In the method of the invention, preferably, the somatic cells are the somatic cells of mammal. More preferably, the mammal is mouse, rat, rabbit, pig, sheep, goat, cattle, monkey or human.

Furthermore, the present invention provides a medium for the preparation of induced pluripotent stem cells, which further comprises lithium salt(s).

Preferably, the lithium salt(s) is/are one or more selected from the group consisting of lithium chloride, lithium carbonate, lithium acetate, lithium bromide, lithium aspartate, lithium γ-linolenatₑ, lithium heparin, lithium sulfate, lithium nitrate, and other chemicals containing lithium ion.

More preferably, the lithium salt is lithium chloride.

Preferably, the concentration of the lithium salt(s) is from 0.1 mM to 40 mM. More preferably, the concentration of the lithium salt(s) is from 0.5 mM to 20 mM. Further more preferably, the concentration of the lithium salt(s) is from 1 mM to 10 mM. Even further more preferably, the concentration of the lithium salt(s) is from 5 mM to 10 mM. Most preferably, the concentration of the lithium salt(s) is 10 mM.

When the concentration of the lithium salt is more than 40 mM, cells will die after long-term cultivation. When the concentration of the lithium salt is less than 0.1 mM, the production efficiency of iPS cells can not be significantly increased.

Preferably, the medium is the mES medium supplemented with lithium salt(s) or the KSR medium supplemented with lithium salt(s).

In the invention, the lithium salt can efficiently enhance the generation of iPS cells. It is demonstrated by flow cytometry analysis that the induction efficiency of the experimental groups, wherein the medium supplemented with the lithium salt is used, is increased by approximately 5 folds (four-factor induction groups) or approximately 60 folds (three-factor induction groups), respectively, when compared to that of the control groups. The reprogramming time was also be shortened by the addition of lithium salt during iPS induction. In experimental groups using medium supplemented with lithium salt, Oct4-GFP⁺ colonies can be detected on the 8^{th} day after infection. While in control groups, Oct4-GFP⁺ colonies are generally detected on 10^{th} days or later after infection. IPS cells induced by supplementing lithium salt have excellent totipotency. IPS clones transduced with four factors or three factors can form chimera mice, wherein germline transmission and birth to offspring are observed in the chimera mice produced with four-factor iPS cells. The present invention provides efficient methods of iPS cell induction, and these methods might promote basic research and clinical application of iPS cells in a substantial way.

### BRIEF DESCRIPTION OF THE DRAWINGS

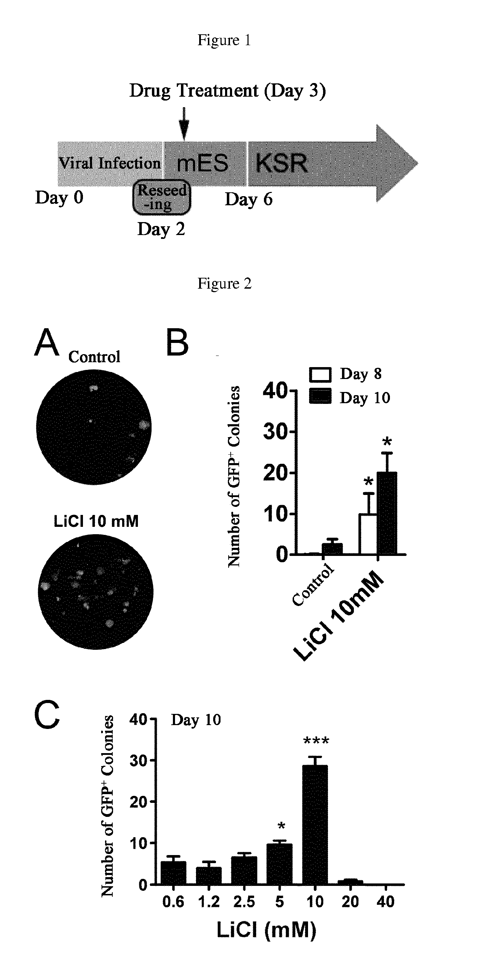

Figure 1 is the flow chart showing the experimental procedures for promoting the production of iPS cells by using the stem cell medium supplemented with lithium salt.
Figure 2 shows the generation efficiency of four-factor (Oct4, Sox2, Klf4 and c-Myc) induced iPS cells is increased and iPS induction time is shortened by using the stem cell medium supplemented with lithium salt. Fig. A is representative images of one well treated with 10 mM LiCl on a 96-well plate wherein many GFP⁺ colonies formed on day 10 after infection. Fig. B is a graph showing statistical data of Oct-GFP⁺ colonies, which indicates that, on day 8 after infection, approximately 10 Oct-GFP⁺ colonies are observed in LiCl treated wells, while GFP⁺ clone could barely be observed in control wells; on day 10 after infection, the number of Oct-GFP⁺ colonies in LiCl treated wells reaches approximately 20 (* indicates p<0.05). Fig. C is a graph showing statistical data of Oct-GFP⁺ colonies by addition of LiCl with different concentrations, wherein significant dose effect can be observed and the most significant effect appears at the concentration of 10 mM (LiCl) (* indicates p<0.05).

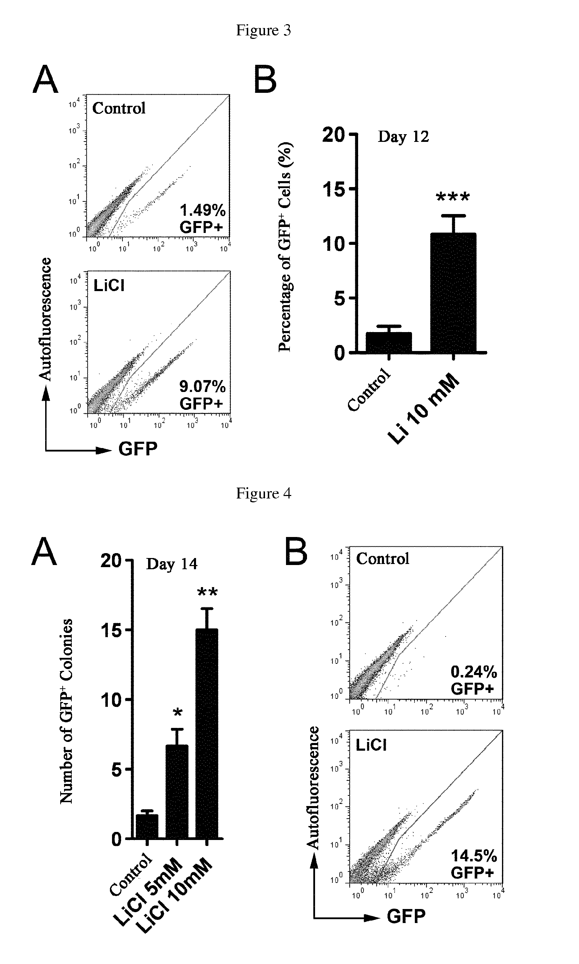

Figure 3 shows the results of flow cytometry analysis, illustrating that the production efficiency of four-factor induced iPS cells is increased by addition of lithium salt. Fig. A is a graph showing the percentage of GFP⁺ cells measured by FACS analysis, wherein cells are trypsinized and collected on day 12 after infection. Fig. B is a graph showing statistical data of three independent experiments (*** indicates p<0.001).
Figure 4 shows the production efficiency of three-factor (Oct4, Sox2, Klf4) induced iPS cells is increased by using the stem cell medium supplemented with lithium salt. Fig. A is a graph showing statistical data of Oct-GFP⁺ colonies, wherein the production efficiency of iPS cells on day 14 after infection can be enhanced by 5 mM or 10 mM of LiCl. Fig. B is a graph showing percentage of GFP⁺ cells measured by flow cytometry, wherein cells are trypsinized and collected on day 16 after infection (* indiciates p<0.05, ** indicates p<0.01).

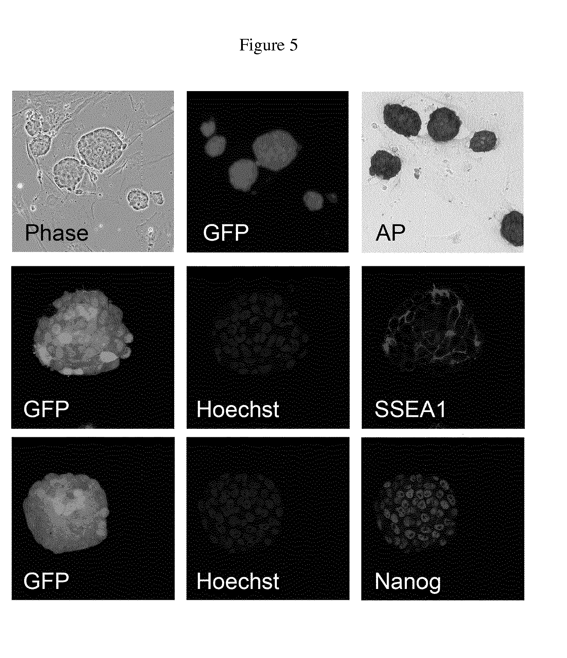

Figure 5 shows results of alkaline phosphatase staining and immunofluorescent staining. Top: showing that iPS cells have morphology similar to embryonic stem cells and strongly express Oct4-GFP, and alkaline phosphatase staining in these cells is positive and uniform. Middle and low: showing immunofluorescent staining of stem cell-specific proteins Nanog and SSEA-1, wherein iPS cells express both stem cell markers.

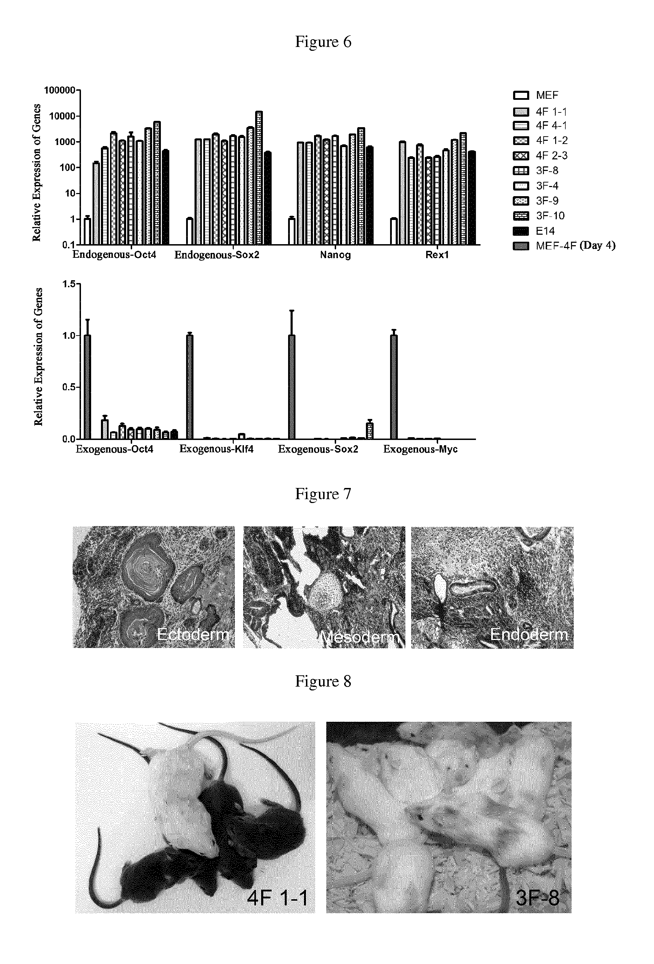

Figure 6 shows an analysis with quantitative PCR, which confirms the expression of stem cell-specific genes and the silencing of exogenous viral genes. Fig. A shows the expression of stem cell-specific genes, wherein mouse embryonic fibroblasts are used as negative controls, and mouse embryonic stem cell line E14 cells are used as positive controls. Both four factors + lithium salt induced iPS clones and three factors + lithium salt induced iPS clones highly express stem cell-specific genes including endogenous Oct4, endogenous Sox2, Nanog and Rex1 genes. Fig. B shows the silencing of exogenous viral genes, wherein mouse embryonic fibroblasts infected with virus for 4 days are used as positive controls, and ES cells are used as negative controls. Both four factors + lithium salt induced iPS clones and three factors + lithium salt induced iPS clones exhibit excellent silencing of exogenous viral genes.
Figure 7 shows results of teratoma formation assays, wherein teratomas are formed from iPS cells induced by four factors + lithium salt. Judging by tissue structure, iPS cells can differentiate into specific structures of three germ layers, which are shown as follows. Left: epidermis-like structure (ectoderm), middle: muscle- and cartilage-like structures (mesoderm), and right: lumen structure of alimentary tract (endoderm).
Figure 8 shows results of chimera formation and germline transmission assays of four factors + lithium salt induced iPS cells. Left: illustrating that four factors + lithium salt induced iPS cells have the capability of germline transmission. Right: illustrating that three factors + lithium salt induced iPS cells have the capability of chimera formation.

### DETAILED DESCRIPTION

### Definition and Technology

The traditional technologies of molecular biology, microbiology, cell biology, immunology and DNA recombination used in the present experiments fall into the scope of technologies in the art, unless otherwise specified. References includes but not limited to: Sambrook, Fritsch and Maniatis, Molecular Cloning: A Laboratory Manual (Third Edition), 2002; Current Protocols in Molecular Biology (F. M. Ausubel et al. (1987)); series of books such as Methods in Enzymology (Academic Press, Inc.); PCR2: A Practical Approach (M. J. MacPherson, B. D. Hames and G. R. Taylor, (1995)); Antibodies, A Laboratory Manual and Animal Cell Culture (R. I. Freshney, (1987)); Handbook of Stem Cells, Vol. 2 (W. French et al.).

Unless otherwise stated, all the terms in this text have the meanings as a person skilled in the art regularly understands. In order to assist the understanding of the present invention, some of the terms used herein are defined as follows:
The term "induced pluripotent stem cells (iPS cells)" used herein refer to those cells obtained from in vitro reprogramming of somatic cells into which stem cell pluripotency factors have been introduced. Such cells are very similar to mouse ES (embryonic stem) cells in many aspects such as cell morphology, growth characteristics, expression of specific gene, manner of DNA methylation and the like, and are very similar to mouse ES cells in aspects such as teratoma formation, chimeric animal formation and germline transmission, etc., under the culture conditions of ES cells.

The said "stem cell pluripotency factors for the induction of somatic cells reprogramming" used herein are key factors in maintaining stem cell pluripotency. Somatic cells can be reprogrammed to stem cells by introducing these factors into the somatic cells. Many factors have been reported to have an ability to induce the reprogramming of somatic cells [14-18]. Preferably, the pluripotency factor(s) is/are one or more selected from the group consisting of Oct4, Sox2 (or Sox1) Klf4 (or Klf2 or KLF5), Nanog, c-Myc (or L-Myc or N-Myc), Lin28 and Esrrb. The aforementioned stem cell pluripotency factors may be derived from any desired species, and mouse stem cell pluripotency factors are preferable.

The said "induction of reprogramming" (sometimes only be simplified as "induction") used herein refers to a process of de-differentiating somatic cells into pluripotency stem cells. Preferably, somatic cells can be induced to de-differentiate into pluripotent stem cells by introducing cDNA of pluripotency factors essential for maintaining stem cell pluripotency into the somatic cells. Among them, preferably, the pluripotency factor(s) is/are one or more selected from the group consisting of Oct4, Sox2 (or Sox1), Klf4 (or Klf2 or Klf5), Nanog, c-Myc (or L-Myc or N-Myc), Lin28 and Esrrb.

The method for introducing cDNA of stem cell pluripotency factors into somatic cells may be performed in various ways, such as viral infection, liposome transfection, electroporation, particle bombardment, transposon-mediated insertion expression, membrane permeable proteins or drug induction, etc. It is preferable that the viral vector containing the cDNA is used for transfection. The viral vectors include lentivirus, retrovirus, adenovirus, and etc. It is preferable to use retroviral vectors (PMX vector).

The said "medium" used herein may be DMEM (Dulbcco's Modified Eagle's Medium) supplemented with 15% fetal bovine serum, 1000U/mL leukaemia inhibitory factor (LIF), L-glutamine, non-essential amino acids, penicillin / streptomycin and β-mercaptoethanol (mES medium), and Knockout DMEM supplemented with 15% serum replacement, 1000U/mL leukaemia inhibitory factor (LIF), L-glutamine, non-essential amino acids, penicillin / streptomycin and β-mercaptoethanol (KSR medium).

The said "reporter gene" used herein is capable of indicating a state in which cells have been transformed into cells similar to the embryonic stem cells. The reporter gene comprises a fluorescent protein sequence or antibiotics resistant gene sequence introduced by using transgenic or homologous recombinant means, which is under the control of the promoter of certain embryonic stem cell-specific genes, and therefore the expression of the fluorescent protein gene or the antibiotic resistance gene is activated when cells come into the state similar to that of embryonic stem cells, so that the cells have certain detectable characteristics. In the embodiment, the used mouse embryonic fibroblasts are OG2 (Oct4-GFP^{+/-}) cells isolated from 13.5-day embryos obtained by mating homozygous male OG2 (Oct4-GFP^{+/+}) mice with female 129 mice.

According to the present invention, the method for detecting cell pluripotency is well applied by a person skilled in the art, including AP staining, cell fluorescence staining, quantitative PCR analysis of the stem cell-specific gene expression and viral gene silencing, *in vivo* teratoma formation analysis, chimera formation analysis and germline transmission detection.

### EXAMPLES

The following examples are provided to illustrate the standard laboratory practices perfomed by the inventors. It is to be understood that the following examples are illustrative only and the present invention is not limited thereto.

Summary of technologies used in the invention.

Each substance mentioned in the description is commercially available from Invitrogen, unless otherwise specified.

### Experimental procedures for the production of retrovirus and the generation of iPS cells are as follows:

Retrovirus were produced by transfecting plat-E cells with pMXs retroviral vectors containing the cDNAs of mouse Oct4, Sox2, Klf4 and c-Myc (purchased from Addgene) using Fugene HD (Roche) according to the manufacturer's protocol. After 48 hours, virus-containing supernatants were collected and filtered. The filtered supernatant was supplemented with 8 mg/L of polybrene, and then was used to infect mouse embryonic fibroblasts. The day when adding virus containing supernatants is counted as "day 0". The fibroblasts infected with the virus were cultured in the mES medium, and iPS colonies were picked from day 14 to the day 18 after infection (four-factor infection experiment) or from day 20 to day 24 after infection (three-factor infection experiment), based on the expression of Oct-GFP and the typical morphology of stem cells.

### Experimental procedure for the quantitation of reprogramming efficiency is as follows:

The main method for calculating the reprogramming efficiency is the counting of the Oct-GFP⁺ cells, including: 1. Using flow cytometry to determine the percentage of Oct-GFP⁺ cells in the cell culture, which were infected with four factors for 10 to 12 days or with three factors for 14 to 16 days, or 2. directly counting the Oct-GFP⁺ colonies in the wells by using fluorescence microscope.

### Experimental procedure for the characterization of iPS cells is as follows:

Alkaline phosphatase staining, fluorescence staining of stem cell marker proteins, identification of expression of pluripotency genes, silencing of viral genes, teratoma formation, chimera formation and germline transmission are performed [19, 20].

### Example 1: the stem cell medium supplemented with lithium salt can promote the formation of four-factor induced iPS cells.

a. Same volumes of the viruses carrying Oct4, Sox2, Klf4 or c-Myc were mixed and added to a total of 180,000 OG2 mouse embryonic fibroblasts in one well of a 6-well plate and cells were cultured in DMEM medium supplemented with 10% fetal bovine serum at 37 °C and 5% CO₂. The day of viral infection was counted as "day 0". On day 2, cells were trypsinized and resuspended in the mES medium, and then seeded at a density of 5,000 cells per well onto a 96-well plate pre-seeded with irradiated mouse embryonic fibroblast feeders. The mES medium supplemented with different concentrations of LiCl (0.6 mM, 1.2 mM, 2.5 mM, 5 mM, 10 mM, 20 mM and 40 mM) were used from day 3. On day 6, the medium was replaced with KSR medium supplemented with different concentrations of LiCl (0.6 mM, 1.2 mM, 2.5 mM, 5 mM, 10 mM, 20 mM and 40 mM). On day 8, the medium was replaced with KSR medium again. The entire process was shown in Figure 1.
b. Using the method described in step a, from day 8, Oct-GFP⁺ colonies were observed and counted under an inverted fluorescence microscope every day, and pictures were also taken with a fluorescence microscope. Figure 2A was representative images of wells in the 96-well plate on day 10, in which there were more GFP⁺ colonies in 10 mM LiCl treated well than in untreated well. Figure 2B showed statistical data of Oct-GFP⁺ colonies. On day 8, GFP⁺ colony could barely be observed in control wells, while approximately 10 GFP⁺ colonies could be observed in 10 mM LiCl treated wells. On day 10, approximately 20 GFP⁺ colonies could be observed in 10 mM LiCl treated wells, with induction efficiency 5 to 6 times of that of control wells'. Figure 2C showed the number of positive colonies generated after the treatment with different concentrations of LiCl (0.6 mM, 1.2 mM, 2.5 mM, 5 mM, 10 mM, 20 mM and 40 mM). Significant dose effect was observed, wherein the most effective dose of LiCl was 10 mM.
c. Using the method described in step a, on day 12, cells were trypsinized and percentage of Oct-GFP⁺ cells was determined by using flow cytometry. Figure 3A was representative FACS plots. Figure 3B showed the statistical data of three independent experiments, demonstrating that the induction efficiency of wells treated with 10 mM LiCl was increased by 5 to 6 times than that of wells without treatment, when using four factors to induce reprogramming.

### Example 2: the stem cell medium supplemented with lithium salt can promote the formation of three-factor induced iPS cells.

a. Same volumes of the viruses carrying Oct4, Sox2 or Klf4 were mixed and added to a total of 180,000 OG2 mouse embryonic fibroblasts in one well of a 6-well plate and cells were cultured in DMEM medium supplemented with 10% fetal bovine serum at 37 °C and 5% CO₂. The day of viral infection was counted as "day 0". On day 2, cells were trypsinized and resuspended in the mES medium, and seeded at a density of 5,000 cells per well onto a 96-well plate pre-seeded with irradiated mouse embryonic fibroblast feeders. The mES medium supplemented with different concentrations of LiCl (0.6 mM, 1.2 mM, 2.5 mM, 5 mM, 10 mM, 20 mM and 40 mM) were used from day 3. On day 6, medium was replaced with KSR medium supplemented with LiCl having different concentrations (0.6 mM, 1.2 mM, 2.5 mM, 5 mM, 10 mM, 20 mM and 40 mM). At day 8, medium was replaced with KSR medium again. The entire process was shown in Figure 1.
b. Using the method described in step a, from day 12, Oct-GFP⁺ colonies were observed and counted under an inverted fluorescence microscope every day, and pictures were also taken with a fluorescence microscope. On day 14, as compared with control groups, the induction efficiency of iPS cells in wells treated with 5 mM or 10 mM LiCl was significantly improved, wherein the induction efficiency of iPS cells in wells treated with 10 mM LiCl could be increased by approximately 7 times, as shown in Figure 4A.
c. Using the method described in step a, on day 16, cells were trypsinized and the percentage of Oct-GFP⁺ cells was determined by using flow cytometry. The percentage of Oct-GFP⁺cells in control groups was only 0.24%, while the percentage of Oct-GFP⁺ cells treated with 10 mM of LiCl in experimental groups reached 14.5%, with an increase of approximately 60 times, as shown in Figure 4B.

### Example 3: iPS cell lines obtained by addition of lithium salt are pluripotent.

a. As described above, mouse embryonic fibroblasts were infected with four factors of Oct4, Sox2, Klf4 and c-Myc or three factors of Oct4, Sox2 and Klf4, and cultured in the stem cell medium supplemented with LiCl. On day 14 after infection (four factors) or on day 20 after infection (three factors), representative colonies were picked out based on morphology and fluorescence expression, and were used to generate homogeneous iPS cell lines through passages.
b. The established iPS cell lines were observed in terms of their morphology and stained to detect stem cell-specific proteins. As shown in Figure 5, iPS cells had characteristic morphology similar to that of embryonic stem cells, strongly expressed Oct-GFP, and were positive in alkaline phosphatase (AP) staining. Immunofluorescent staining of the iPS cells was carried out by using antibodies against stem cell-specific proteins, and results demonstrated that iPS cells expressed stem cell-specific proteins Nanog and SSEA-1.
c. Feeder cells were removed by using differential adhesion method prior to extraction of RNA from iPS cells. Total RNA was extracted using Trizol (Invitrogen) reagent according to the manufacturer's protocol. Reverse transcription was performed using PrimeScript^{™} kit (Takara co.). Real-time PCR was performed by using JumpStart^{™} TaqReady Mix^{™} kit (Sigma co.) and Mx 3000P thermal cycler (ABI co.). Common PCR conditions were used according to the manufacturer's protocol. Primers for identifying iPS specific genes were listed in table 1.

**[Table 1]**

| Primer Name (F: forward; R: reverse) | Primer Sequence |
|---|---|
| endogenous-Oct4 F | TAGGTGAGCCGTCTTTCCAC |
| endogenous-Oct4 R | GCTTAGCCAGGTTCGAGGAT |
| Nanog F | CTCAAGTCCTGAGGCTGACA |
| Nanog R | TGAAACCTGTCCTTGAGTGC |
| endogenous-Sox2 F | AGGGCTGGGAGAAAGAAGAG |
| endogenous-Sox2 R | CCGCGATTGTTGTGATTAGT |
| Rex1 F | GACGAAGCAAGAGAAGAG |
| Rex1 R | CGATAAGACACCACAGTAC |
| exogenous-Sox2 F | GGGTGGACCATCCTCTAGAC |
| exogenous-Sox2 R | GGGCTGTTCTTCTGGTTG |
| exogenous-Klf4 F | GGGTGGACCATCCTCTAGAC |
| exogenous-Klf4 R | GCTGGACGCAGTGTCTTCTC |
| exogenous-cMyc F | GGGTGGACCATCCTCTAGAC |
| exogenous-cMyc R | CCTCGTCGCAGATGAAATAG |
| exogenous-Oct4 F | GCTTGGATACACGCCGC |
| exogenous-Oct4 R | TTCATGTCCTGGGACTCCTC |

As shown in Figure 6A, iPS cells obtained by using the method of the invention expressed high-level of pluripotency factors including endogenous Oct4, endogenous Sox2, Nanog and Rex1, and their expression levels were comparable to those of embryonic stem cell line E14. On the contrary, expression of these factors was not detectable in the original mouse embryonic fibroblasts. Meanwhile, as shown in Figure 6B, iPS cells obtained by using the method of the invention could silence the viral genes fairly well, demonstrating that cells were completely reprogrammed into stem cells.
d. Mouse four-factor induced iPS cells obtained by adding lithium salt were trypsinized, and approximately 1 x 10⁶ iPS cells were resuspended in 200 µl of mES medium and injected into the muscle of femoribus internus in NOD-SCID mice. 4-5 weeks later, mice were sacrificed and teratomas were embedded in paraffin, sectioned, HE stained and analyzed. As shown in Figure 7, tissues originating from three germ layers were found in sections of teratomas formed from iPS cells, which including epidermis-like tissue representing ectoderm, muscle- and cartilage-like tissues representing mesoderm, and lumen-like tissues of alimentary tract representing endoderm. Thus, similar to mouse embryonic stem cells, mouse four-factor induced iPS cells obtained by adding lithium salt were pluripotent and could contribute to different types of cells of the three germ layers.
e. Mouse four-factor induced iPS cells obtained by adding lithium salt were injected into the blastocysts of ICR mouse and thus mixed with the mouse embryonic stem cells in the blastocyst. The blastocysts were then transplanted into the uterus of the pseudopregnant female ICR mice for normal develop. The coat color of the pups was observed to judge whether a chimera was formed. If chimeras were detected, they were mated with white ICR mice and the coat color of the offspring animals was observed to judge whether the germline transmission occured. As shown in Figure 8, both three-factor and four-factor iPS cells could produce chimera mice. And black mice appeared in the offsprings of the chimera mice generated by four-factor iPS cells. These results confirmed that the iPS cells obtained by adding lithium salt did contribute to the germline, demonstrating that these cells are pluripotent and very similar to embryonic stem cells.

In short, iPS cells can be efficiently generated by using the medium supplemented with lithium salt in the present invention. It is demonstrated by the results from flow cytometry analysis that the induction efficiency of experimental groups treated with lithium salt can be increased by 5 to 6 times (four-factor induction assay) and approximately 60 times (three-factor induction assay) than that of the control groups. The reprogramming time can also be shortened by adding lithium salt during iPS induction. Oct4-GFP⁺ colonies can be detected on day 8 after infection in experimental groups treated with lithium salt, while Oct4-GFP⁺ colonies have not been found until day 10 in the control groups. IPS cells induced by using lithium salt have excellent pluripotency. IPS clones transduced with four factors or three factors can form chimera mice, wherein germline transmission and birth to offsprings are observed in chimera mice generated with four-factor iPS cells. The present invention provides efficient method for inducing iPS cells, and the method might substantially promote the basic research and clinical application of iPS cells.

The contents shown above is only the preferred examples of the present invention, obviously, they can not define the scope of the rights of the present invention, therefore equivalent changes made according to claims claimed in the present invention still fall into the scope of the present invention.

### References:

1. Evans, M.J. and M.H. Kaufman, Establishment in culture of pluripotential cells from mouse embryos. Nature, 1981. 292(5819): p. 154-6.
2. Thomson, J.A., et al., Embryonic stem cell lines derived from human blastocysts. Science, 1998. 282(5391): p. 1145-7.
3. Takahashi, K. and S. Yamanaka, Induction of pluripotent stem cells from mouse embryonic and adult fibroblast cultures by defined factors. Cell, 2006. 126(4): p. 663-76.
4. Meissner, A., M. Wernig, and R. Jaenisch, Direct reprogramming of genetically unmodified fibroblasts into pluripotent stem cells. Nat Biotechnol, 2007. 25(10): p. 1177-81.
5. Okita, K., T. Ichisaka, and S. Yamanaka, Generation of germline-competent induced pluripotent stem cells. Nature, 2007. 448(7151): p. 313-7.
6. Wernig, M., et al., In vitro reprogramming of fibroblasts into a pluripotent ES-cell-like state. Nature, 2007. 448(7151): p. 318-24.
7. Park, I.H., et al., Reprogramming of human somatic cells to pluripotency with defined factors. Nature, 2008. 451(7175): p. 141-6.
8. Yu, J., et al., Induced pluripotent stem cell lines derived from human somatic cells. Science, 2007. 318(5858): p. 1917-20.
9. Zhao, X.Y., et al., iPS cells produce viable mice through tetraploid complementation. Nature, 2009. 461(7260): p. 86-90.
10. Nakagawa, M., et al., Generation of induced pluripotent stem cells without Myc from mouse and human fibroblasts. Nat Biotechnol, 2008. 26(1): p. 101-6.
11. Li, Y, et al., Generation of iPSCs from mouse fibroblasts with a single gene, Oct4, and small molecules. Cell Res, 2010.
12. Stadtfeld, M., et al., Induced pluripotent stem cells generated without viral integration. Science, 2008. 322(5903): p. 945-9.
13. Zhou, H., et al., Generation of induced pluripotent stem cells using recombinant proteins. Cell Stem Cell, 2009. 4(5): p. 381-4.
14. Esteban, M.A., et al., Vitamin C enhances the generation of mouse and human induced pluripotent stem cells. Cell Stem Cell, 2010. 6(1): p. 71-9.
15. Ichida, J.K., et al., A small-molecule inhibitor of tgf-Beta signaling replaces sox2 in reprogramming by inducing nanog. Cell Stem Cell, 2009. 5(5): p. 491-503.
16. Shi, Y, et al., Induction of pluripotent stem cells from mouse embryonic fibroblasts by Oct4 and Klf4 with small-molecule compounds. Cell Stem Cell, 2008. 3(5): p. 568-74.
17. Maherali, N. and K. Hochedlinger, Tgfbeta signal inhibition cooperates in the induction of iPSCs and replaces Sox2 and cMyc. Curr Biol, 2009. 19(20): p. 1718-23.
18. Mali, P., et al., Butyrate greatly enhances derivation of human induced pluripotent stem cells by promoting epigenetic remodeling and the expression of pluripotency-associated genes. Stem Cells, 2010. 28(4): p. 713-20.
19. Yamanaka, S., Strategies and new developments in the generation of patient-specific pluripotent stem cells. Cell Stem Cell, 2007. 1(1): p. 39-49.
20. Evans, M.J., et al., The ability of EK cells to form chimeras after selection of clones in G418 and some observations on the integration of retroviral vector proviral DNA into EK cells. Cold Spring Harb Symp Quant Biol, 1985. 50: p. 685-9.

## Claims

1. A method for preparing induced pluripotent stem cells, which comprises steps as follows:
step 1: introducing one or more stem cell pluripotency factor(s) into somatic cells;
step 2: culturing the somatic cells, into which the stem cell pluripotency factors has been introduced in the step 1, by using medium supplemented with lithium salt(s); and
step 3: identifying and characterizing the induced pluripotent stem cell clones.

2. The method according to claim 1, further comprising a step of introducing a reporter gene into the somatic cells to indicate the generation and production efficiency of the induced pluripotent stem cells.

3. The method according to claim 2, wherein the reporter gene is Oct4-GFP or Nanog-GFP, and preferably, the reporter gene is Oct4-GFP.

4. The method according to claim 1, wherein in the step 1 the stem cell pluripotency factor(s) is/are one or more selected from the group consisting of Oct4, Sox2, Sox1, Klf4, Klf2, Klf5, Nanog, c-Myc, L-Myc, N-Myc, Lin28 and Esrrb.

5. The method according to claim 4, wherein in the step 1 the stem cell pluripotency factors are Oct4, Sox2, Klf4 and c-Myc, or Oct4, Sox2 and Klf4.

6. The method according to claim 1, wherein in the step 2 the lithium salt(s) is/are one or more selected from the group consisting of lithium chloride, lithium carbonate, lithium acetate, lithium bromide, lithium aspartate, lithium γ-linolenatₑ, lithium heparin, lithium sulfate, lithium nitrate and other chemicals containing lithium ion.

7. The method according to claim 1, wherein in the step 2 the lithium salt is lithium chloride.

8. The method according to claim 1, wherein in the step 2 the concentration of the lithium salt(s) is from 0.1 mM to 40 mM, preferably is from 0.5 mM to 20 mM, more preferably is from 5 mM to 10 mM, and most preferably is 10 mM.

9. The method according to claim 1, wherein in the step 2 the medium is mES medium or KSR medium, the mES medium is DMEM supplemented with 15% fetal bovine serum, 1000U/mL leukaemia inhibitory factor, L-glutamine, non-essential amino acid, penicillin/streptomycin and β-mercaptoethanol, and the KSR medium is Knockout DMEM supplemented with 15 % serum replacement, 1000U/mL leukaemia inhibitory factor, L-glutamine, non-essential amino acid, penicillin/streptomycin and β-mercaptoethanol.

10. The method according to claim 1, wherein the step 2 further comprises:
mouse embryonic fibroblasts, into which four factors of Oct4, Sox2, Klf4 and c-Myc or three factors of Oct4, Sox2 and Klf4 have been introduced in the Step 1, are trypsinized, seeded onto feeder cells and cultured by using the mES medium on day 2, on day 3 the medium is replaced with the mES medium supplemented with lithium salt, on day 6 the medium is replaced with the KSR medium supplemented with lithium salt, and on day 8 the medium is replaced with the KSR medium, after that colonies with excellent stem cell-like morphology or Oct4-GFP⁺ colonies are selected and used for passage.

11. The method according to any one of previous claims, wherein the somatic cells are the somatic cells of mammals.

12. The method according to claim 11, wherein the mammal is selected from mouse, rat, rabbit, pig, sheep, goat, cattle, monkey or human.

13. A medium for preparing induced pluripotent stem cells, which further comprises lithium salt(s).

14. The medium according to claim 13, wherein the lithium salt(s) is/are one or more selected from the group consisting of lithium chloride, lithium carbonate, lithium acetate, lithium bromide, lithium aspartate, lithium γ-linolenatₑ, lithium heparin, lithium sulfate, lithium nitrate and other chemicals containing lithium ion.

15. The medium according to claim 13, wherein the lithium salt is lithium chloride.

16. The medium according to claim 13, wherein the concentration of the lithium salt(s) is from 0.1 mM to 40 mM, preferably is from 0.5 mM to 20 mM, more preferably is from 5 mM to 10 mM, and most preferably is 10 mM.

17. The medium according to claim 13, wherein the medium is the mES medium supplemented with lithium salt(s) or the KSR medium supplemented with lithium salt(s).
